(19) Europäisches Patentamt
European Patent Office
Office européen des brevets

(11) EP 4 389 228 A1

(12) EUROPEAN PATENT APPLICATION

(43) Date of publication:
**26.06.2024 Bulletin 2024/26**

(21) Application number: **22383254.4**

(22) Date of filing: **21.12.2022**

(51) International Patent Classification (IPC):
***A61Q 11/00*** *(2006.01)* ***A61K 9/00*** *(2006.01)*
***A61K 8/73*** *(2006.01)* ***A61K 8/58*** *(2006.01)*
***A61K 8/34*** *(2006.01)* ***A61K 8/60*** *(2006.01)*
***A61K 8/365*** *(2006.01)* ***A61K 8/92*** *(2006.01)*
***A61K 8/02*** *(2006.01)* ***A61K 8/04*** *(2006.01)*
***A61K 47/10*** *(2017.01)* ***A61K 47/12*** *(2006.01)*
***A61K 47/24*** *(2006.01)* ***A61K 47/26*** *(2006.01)*
***A61K 47/38*** *(2006.01)* ***A61K 47/44*** *(2017.01)*
***A61P 1/02*** *(2006.01)*

(52) Cooperative Patent Classification (CPC):
**A61Q 11/00; A61K 8/0216; A61K 8/04; A61K 8/345; A61K 8/347; A61K 8/365; A61K 8/58; A61K 8/60; A61K 8/731; A61K 8/922; A61K 9/0053; A61K 47/10; A61K 47/12; A61K 47/24; A61K 47/26;** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Lacer, S.A.U.**
**08290 Cerdanyola del Vallès, Barcelona (ES)**

(72) Inventors:
- **DEL CASTILLO NIETO, Juan Carlos**
  **E-08290 Cerdanyola del Vallès, Barcelona (ES)**
- **FERRÀ DOMINGO, Lourdes**
  **E-08290 Cerdanyola del Vallès, Barcelona (ES)**
- **FERRER SISO, Alicia**
  **E-08290 Cerdanyola del Vallès, Barcelona (ES)**
- **MARTÍN HERRERO, Cristina**
  **E-08290 Cerdanyola del Vallès, Barcelona (ES)**
- **MATA MOLINER, Montserrat**
  **E-08290 Cerdanyola del Vallès, Barcelona (ES)**
- **MIRA OTAL, Francisco Javier**
  **E-08290 Cerdanyola del Vallès, Barcelona (ES)**
- **RODRÍGUEZ AMIGO, Beatriz**
  **E-08290 Cerdanyola del Vallès, Barcelona (ES)**

(74) Representative: **ABG Intellectual Property Law, S.L.**
**Avenida de Burgos, 16D**
**Edificio Euromor**
**28036 Madrid (ES)**

# (54) ORAL CARE COMPOSITION CONTAINING CELLULOSIC POLYMERS

(57) Oral care composition comprising:
- a cellulosic polymer,
- o-cymen-5-ol, and
- chlorhexidine or salts thereof.

Said composition for use in the treatment and/or prevention of dental plaque formation, caries formation, calculus formation, halitosis, gingivitis, periodontitis, peri-implant mucositis and/or periimplantitis.

EP 4 389 228 A1

(52) Cooperative Patent Classification (CPC): (Cont.)
**A61K 47/38; A61K 47/44; A61P 1/02;**
A61K 2800/59; A61K 2800/92

## Description

### FIELD OF THE INVENTION

The invention relates to the field of oral care compositions, in particular to mouthwashes, tooth gels and toothpastes comprising cellulosic polymeric binders such as hydroxyethylcellulose and hydroxypropyl methyl cellulose and antimicrobial agents such as chlorhexidine and *o*-cymen-5-ol.

### BACKGROUND OF THE INVENTION

Oral hygiene is the practice of keeping the mouth clean, and is considered to be the best means of prevention of cavities (dental caries), gingivitis, periodontitis, and other dental and oral disorders such as bad breath (halitosis). Oral hygiene is necessary for all persons to maintain the health of their teeth and mouth. There are three key steps to regular dental health and maintenance: brushing, flossing and rinsing with a mouthwash. Teeth brushing involves the use of a toothpaste in conjunction with a toothbrush to help remove food debris and dental plaque. Mouthwashes are used for cleaning and freshening the mouth and for oral hygiene. Antiseptic and anti-plaque compounds are present in mouthwashes and toothpaste to kill germs that cause plaque, gingivitis, and bad breath.

Sensory-related aspects are an important contribution to consumers' acceptance and preference of oral care products. Examples of relevant sensory-related aspects for oral care products are appearance, sensory smoothness and granularity, and taste.

Mucoadhesivity of oral care compositions is an interesting property of oral care compositions which may enhance the effectivity of said compositions by increasing the time they remains in the area to be treated thereby prolonging the contact of the active components in the area upon use (see for example Mašková E. et al, Journal of Controlled Release, V. 324, 10 August 2020, pp 695-727). Cellulosic polymers have been used in the past to enhance bioadhesivity of compositions to mucosae (see WO 2005/014047 A1).

Antimicrobial agents are widely used in oral healthcare products, such as toothpastes and mouthwashes, to confer antimicrobial effect against different bacteria, which helps control the development of dental plaque, calculus formation, bleeding gums, and malodour. They may also help reduce the breath concentrations of volatile sulphur compounds (VSC), which are associated with halitosis, as well as having direct and indirect inhibitory effects on anaerobe mediated-VSC production. Therefore, the inclusion of antimicrobial compounds in oral care products is highly desirable.

One type of antibacterial compounds which have been proposed for use in oral care products are isopropylmethylphenols. These compounds penetrate deep-inside plaque and show high bactericidal effect [Park, Y.-D. et al., Int. J. Clin. Prev. Dent., 2010, 6(2), 55-61]. In particular o-cymen-5-ol has been proposed as an antibacterial to be used oral care products [Pizzey, R. L., Int. Dent. J., 2011, 61 (Suppl. 3), 33-40].

Chlorhexidine (CHX) and its salts have also found widespread use as a gold standard antimicrobial agent for the reduction of both plaque and gingivitis (Najafi MH et al., Dent Res J 2012;9:305-8).

### SUMMARY OF THE INVENTION

The present inventors have found that the addition of chlorhexidine to oral compositions comprising cellulosic polymers tends to decrease the mucoadhesivity of said oral compositions but that further addition of o-cymen-5-ol to said compositions, which comprise both chlorhexidine or salts thereof and cellulosic polymers, mitigates the negative impact of chlorhexidine (or its salts) or even increases the mucoadhesivity of the compositions in comparison with those comprising cellulosic polymers but neither chlorhexidine (or its salts) nor *o*-cymen-5-ol.

Thus, in a first aspect, the present invention relates to an oral care composition comprising:

- a cellulosic polymer,
- *o*-cymen-5-ol, and
- chlorhexidine or salts thereof.

In a second aspect, the present invention relates to an oral care composition as defined in the first aspect for use in the treatment and/or prevention of dental plaque formation, caries formation, calculus formation, halitosis, gingivitis, periodontitis, peri-implant mucositis and/or periimplantitis.

In a third aspect, the present invention relates to the use of an oral care composition as defined in the first aspect for the manufacture of a medicament for treating and/or preventing dental plaque formation, caries formation, calculus formation, halitosis, gingivitis, periodontitis, peri-implant mucositis and/or periimplantitis.

In a fourth aspect, the present invention relates to a method of treatment and/or prevention of dental plaque formation, caries formation, calculus formation, halitosis, gingivitis, periodontitis, peri-implant mucositis and/or periimplantitis comprising contacting a surface of the oral cavity with the oral care compositions as defined in the first aspect.

In a fifth aspect, the present invention relates to the use of o-cymen-5-ol to increase the mucoadhesivity of compositions comprising a cellulosic polymer, in particular of compositions comprising both a cellulosic polymer and chlorhexidine or salts thereof.

## DETAILED DESCRIPTION OF THE INVENTION

In a first aspect, the present invention relates to an oral care composition comprising:

- a cellulosic polymer,
- *o*-cymen-5-ol, and
- chlorhexidine or salts thereof.

The compositions of the invention are "oral care" compositions, which means that they are intended for topical use in the mouth, so any component present in the composition needs to be orally acceptable, that is, safe for topical use in the mouth in the amounts and concentrations provided.

The term "o-cymen-5-ol" refers to 4-isopropyl-3-methylphenol of formula (I).

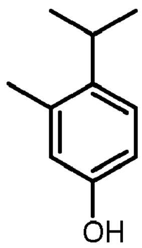

(I)

In a particular embodiment of the present invention *o*-cymen-5-ol is present at a concentration of from 0.005 to 1 wt%, preferably from 0.01 to 0.5 wt%, more preferably from 0.02 to 0.2 wt%, most preferably from 0.05 to 0.1 wt% with respect to the total weight of the composition.

The term "cellulosic polymer" is used in the present invention to designate any one of the following: hydroxyethylcellulose, carboxymethylcellulose (carmellose), hydroxypropyl methyl cellulose, hydroxypropylcellulose, ethyl cellulose, methylcellulose and salts thereof (e.g., carmellose sodium).

In a particular embodiment of the present invention the cellulosic polymer is selected from the group consisting of hydroxyethyl cellulose and hydroxypropyl methyl cellulose.

In a particular embodiment of the present invention the cellulosic polymer is selected from the group consisting of hydroxypropyl methylcellulose and hydroxyethyl cellulose.

In a particular embodiment of the present invention the cellulosic polymer is present at a concentration of from 0.05 to 4 wt% with respect to the total weight of the composition.

The term "chlorhexidine" is used in the present invention to designate the compound of formula:

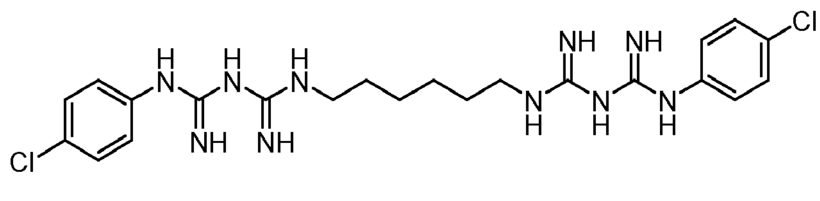

When the present invention refers to salts of chlorhexidine it is to be understood that any physiologically acceptable salt of the compound is included. These include, inter alia, the following: chlorhexidine gluconate, chlorhexidine digluconate, chlorhexidine acetate, chlorhexidine diacetate, chlorhexidine dihydrochloride, chlorhexidine phosphanilate (wherein phosphanilic acid is the compound shown below:

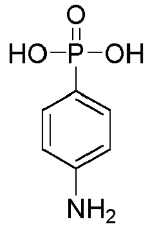

).

In a preferred embodiment chlorhexidine is used in the form of its salts, in particular chlorhexidine gluconate, chlorhexidine digluconate, chlorhexidine acetate, chlorhexidine diacetate, chlorhexidine dihydrochloride, more preferably chlorhexidine digluconate.

In a particular embodiment of the present invention chlorhexidine or the salts thereof are present at a concentration of from 0.05 to 0.5 wt% (expressed as chlorhexidine free base) with respect to the total weight of the composition.

In a particular embodiment of the present invention the oral care compositions do not comprise any zinc salt having a solubility in water higher than $3.0 \cdot 10^4$ mg/L at 25 °C. Examples of zinc salts having a solubility in water higher than $3.0 \cdot 10^4$ mg/L at 25 °C are those belonging to the group consisting of zinc chloride, zinc sulphate such as the heptahydrate, zinc gluconate, zinc citrate, zinc acetate and mixtures thereof; preferably zinc chloride, zinc sulphate and zinc gluconate and mixtures thereof; more preferably zinc chloride, zinc gluconate and zinc acetate and mixtures thereof; more preferably zinc chloride, zinc gluconate and mixtures thereof; even more preferably zinc chloride.

In a particular embodiment of the present invention the oral care compositions do not comprise any glycyrrhizin derivative.

Examples of glycyrrhizin derivatives are those belonging to the groups consisting of glycyrrhizinic acid, glycyrrhetinic acid, salts thereof, and mixtures thereof.

"Glycyrrhizinic acid" or "glycyrrhizic acid" has the chemical structure depicted below.

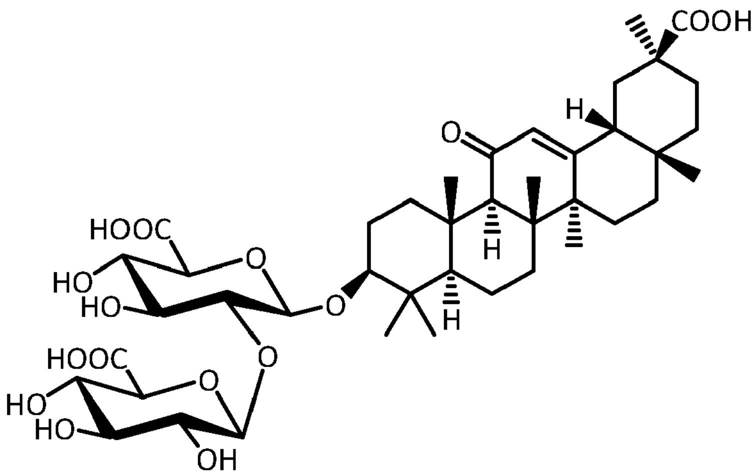

(II)

Glycyrrhizinic acid has three acid groups which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhizinic acid are potassium, sodium and ammonium. Examples of glycyrrhizinic acid salts are dipotassium glycyrrhizinate, diammonium glycyrrhizinate, disodium glycyrrhizinate, monoammonium glycyrrhizinate, monosodium glycyrrhizinate, and monopotassium glycyrrhizinate, preferably dipotassium glycyrrhizinate.

"Glycyrrhetinic acid" or "enoxolone" has the chemical structure depicted below.

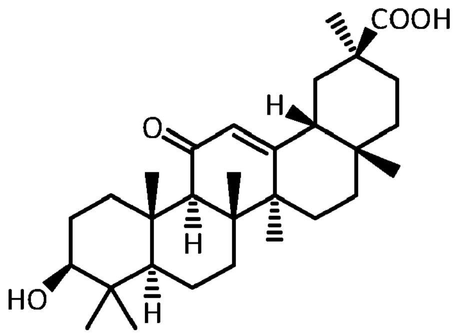

(II)

Glycyrrhetinic acid has one acid group which may form salts with a cation, including organic cations (such as ammonium) as well as inorganic cations (such as alkali metals, e.g. sodium and potassium; and alkaline earth metals, e.g. calcium and magnesium). Preferred cations for forming salts with glycyrrhetinic acid are potassium, sodium and ammonium. Examples of glycyrrhetinic acid salts are potassium glycyrrhetinate, ammonium glycyrrhetinate, and sodium glycyrrhetinate.

In a particular embodiment of the present invention the oral care compositions do not comprise hydroxyacetophenone.

In a particular embodiment of the present invention the oral care compositions comprise water, particularly in an amount of at least 10 wt%, at least 15 wt%, at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

In a particular embodiment of the present invention the oral care compositions further comprise one or more ingredients selected from the group consisting of fluoride sources, surfactants, co-solvents, humectants, sweeteners, binders, mouth-feel agents, abrasives, desensitizing agents, other oral care active ingredients, pH modifying agents, other antimicrobial agents, chelating agents, flavoring agents, and colorants.

In a particular embodiment of the present invention the oral care compositions are in the form of a mouthwash, a tooth gel or a toothpaste.

In a particular embodiment of the present invention the oral care compositions are in the form of a mouthwash which comprises:

- 0.005 to 1 wt% of o-cymen-5-ol,
- 0.05 to 4 wt% of a cellulosic polymer selected from the group consisting of hydroxypropyl methyl cellulose and hydroxyethyl cellulose,
- 0.05 to 4 wt% of chlorhexidine or a salt thereof (expressed as chlorhexidine free base)
- at least 70 wt% of water,
- optionally 5 to 10 wt% of propylene glycol,
- optionally 1 to 10 wt% of glycerin, and
- optionally 1 to 5 wt% PEG40 hydrogenated castor oil,

wherein the wt% is expressed with respect to the total weight of the composition.

In a particular embodiment the oral care compositions are in the form of a tooth gel or toothpaste which comprises:

- - 0.005 to 1 wt% of o-cymen-5-ol,

- 0.05 to 4 wt% of a cellulosic polymer selected from the group consisting of hydroxypropyl methyl cellulose and hydroxyethyl cellulose,

- 0.05 to 4 wt% of chlorhexidine or a salt thereof (expressed as chlorhexidine free base)

- - 10 to 75 wt% of water,
- - optionally 5 to 55 wt% of sorbitol,
- - optionally 0.1 to 1.5 wt% xanthan gum,
- - optionally 0.5 to 10 wt% of propylene glycol, and
- - optionally 1 to 35 wt% of glycerin,

wherein the wt% is expressed with respect to the total weight of the composition.

The oral care compositions of the invention also comprise water. Preferably, water is present in an amount of at least 10 wt%, at least 15 wt%, at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

In a preferred embodiment, the oral care composition of the invention also comprise one or more ingredients selected from the group consisting of fluoride sources, surfactants, co-solvents, humectants, sweeteners, binders, mouthfeel agents, abrasives, desensitizing agents, other oral care active ingredients, pH modifying agents, chelating agents, other antimicrobial agents, flavoring agents, and colorants.

Fluoride ion sources include sodium fluoride, potassium fluoride, stannous fluoride, potassium monofluorophosphate, sodium monofluorophosphate, ammonium monofluorophosphate, sodium fluorosilicate, ammonium fluorosilicate, amine fluoride such as (N'-octadecyltrimethylendiamine-N,N,N'-tris(2-ethanol)-dihydrofluoride), ammonium fluoride, and combinations thereof. Preferably, the oral care composition of the invention may also contain a source of fluoride ions or fluorine-providing ingredient in amounts sufficient to supply 50 to 5000 ppm fluoride ion, e.g., from 100 to 1000 ppm fluoride ion. Fluoride ion sources may be added to the compositions of the invention at a level in the range of 0.001 wt% to 10 wt%, e.g., from 0.003 wt% to 5 wt%, or from 0.01 wt% to 1.2 wt%, wherein the wt% is expressed with respect to the total weight of the composition. However, it is to be understood that the weights of fluoride salts to provide the appropriate level of fluoride ion will obviously vary based on the weight of the counter ion in the salt, and one of skill in the art may readily determine such amounts. Preferred fluoride ion sources are sodium fluoride and sodium monofluorophsphate.

The oral care compositions of the present invention may optionally comprise a surfactant. The term "surfactant" refers to compounds that are capable of lowering the surface tension. Suitable surfactants include non-ionic, anionic, cationic and amphoteric surfactants. Any orally acceptable surfactant can be used.

Examples of non-ionic surfactants are block copolymers such as ethylene oxide and propylene oxide block copolymers (e.g. poloxamers), ethoxylated hydrogenated castor oils, polyoxyethylene sorbitan esters (e.g. polysorbates), fatty alcohol ethoxylates, alkylphenol ethoxylates, tertiary amine oxides, tertiary phosphine oxides, dialkylsulfoxides and the like. In a particular embodiment, the non-ionic surfactant is selected from ethylene oxide and propylene oxide block copolymers (e.g. poloxamers), ethoxylated hydrogenated castor oils, and mixtures thereof; preferably poloxamer 407, PEG 40 hydrogenated castor oil and mixtures thereof.

Examples of anionic surfactants are alkyl and alkenyl sulfates and their alkyl-ether derivatives, sarcosinates, isethionates and taurates, such as sodium lauryl sulfate, sodium coconut monoglyceride sulfonate, sodium lauryl sarcosinate, sodium lauryl isethionate, sodium laureth carboxylate, salts of $C_{8}$-$_{20}$alkyl sulfates (such as sodium $C_{14}$-$C_{16}$ alkyl suflates), salts of $C_{8}$-$_{20}$alkenyl sulfates (such as sodium $C_{14}$-$C_{16}$ alkenyl sulfates), and sodium dodecyl benzenesulfonate; preferably sodium lauryl sarcosinate and sodium salts of $C_{8}$-$_{20}$alkyl sulfates (such as sodium $C_{14}$-$C_{16}$ alkyl suflates) and sodiumc salts of $C_{8}$-$_{20}$alkenyl sulfates (such as sodium $C_{14}$-$C_{16}$ alkenyl sulfates).

Examples of cationic surfactants are lauryl trimethylammonium chloride, cetyl pyridinium chloride, cetyl trimethylammonium bromide, diisobutylphenoxyethyl dimethylbenzylammonium chloride, coconut alkyltrimethylammonium nitrite, cetyl pyridinium fluoride and mixtures thereof.

Examples of amphoteric surfactants are myristyl betaine, palmityl betaine, lauryl betaine, cetyl betaine, stearyl betaine, lauramidopropyl betaine, cocoamidoethyl betaine, cocoamidopropyl betaine and the like; preferably cocamidopropylbetaine.

Preferred surfactants include polyoxyethylene hydrogenated castor oils, poloxamers (polypropylene glycol-polyethylene glycol block copolymers), salts of $C_{8}$-$_{20}$alkyl sulfates, salts of $C_{8}$-$_{20}$alkenyl sulfates, sodium lauryl sarcosinate, and cocoamidopropyl betaine.

One or more surfactants are optionally present in a total amount in the range of from 1 wt% to 70 wt%, wherein the wt% is expressed with respect to the total weight of the composition.

In addition to water, the oral care compositions of the invention may comprise one or more co-solvents such as glycerin, propylene glycol and/or ethanol. One or more co-solvents are optionally present in a total amount in the range of from 5 wt% to 20 wt%, wherein the wt% is expressed with respect to the total weight of the composition.

The oral care compositions of the present invention may optionally comprise a humectant, which is a substance that holds and retains moisture. Any orally acceptable humectant can be used, such as sugar alcohols, e.g. sorbitol and, and low molecular weight PEGs, such as PEG40. Most humectants also function as sweeteners. One or more humectants are optionally present in a total amount in the range of from 1 wt% to 70 wt%, wherein the wt% is expressed with respect to the total weight of the composition.

In addition to the humectants described above and to the glycyrrhizin derivatives described above, which also function as sweeteners (i.e. substances capable of providing a sweet taste), the oral care compositions of the present invention may optionally comprise a sweetener which is not a humectant. Examples of this type of sweeteners include artificial sweeteners such as xylitol, erythritol, sucralose, saccharin, acesulfame, neotam, neohesperidin, cyclamate,

thaumatin, stevioside. These sweeteners (which are neither humectants not glycyrrhizin derivatives) may be present in amounts in the range of 0.001 wt% to 2 wt%, wherein the wt% is expressed with respect to the total weight of the composition.

The oral care compositions of the invention may contain a binder, which provides cohesion to the composition. Any conventional binder may be utilized, such as, carboxyvinyl polymers, carrageenans, starches, natural gums such as xanthan gum, karaya, gum arabic and tragacanth, and chitosan. One or more binders may be present in a total amount in the range 0.1 wt% to 1.5 wt%, wherein the wt% is expressed with respect to the total weight of the composition.

Mouth-feel agents that may be present in the oral care compositions of the invention and refer to materials which impart a desirable texture or other feeling during use of the composition. Such agents include bicarbonate salts, which may impart a clean feel to teeth and gums due to effervescence and release of carbon dioxide. Any orally acceptable bicarbonate can be used, such as alkali metal bicarbonates e.g. sodium and potassium bicarbonates, ammonium bicarbonate, and mixtures thereof. One or more bicarbonate salts are optionally present in a total amount in the range of 0.1 wt% to 5 wt%, wherein the wt% is expressed with respect to the total weight of the composition.

The oral care compositions of the present invention may also include an abrasive, which removes material from the teeth and gum surfaces and aids in mechanical tooth cleaning. The abrasive may be a silica abrasive such silica, sodium metaphosphate, potassium metaphosphate, tricalcium phosphate, dihydrated dicalcium phosphate, aluminum silicate, calcined alumina, bentonite or other siliceous materials, sodium chloride, or combinations thereof. One or more abrasives may be present in a total amount in the range from 10 wt% to 30 wt%, preferably from 15 wt% to 20 wt% %, wherein the wt% is expressed with respect to the total weight of the composition.

The oral care composition of the present invention may also comprise a desensitizing agents, which treat hypersensitivity by blocking dentin tubules when applied to a tooth. Examples of desensitizing agents are potassium salts such as potassium nitrate, potassium chloride, potassium bicarbonate, potassium citrate, and potassium oxalate. Such agents may be added in effective amounts, e.g., in a total amount typically in the range from 0.01 wt % to 5 wt%, wherein the wt% is expressed with respect to the total weight of the composition.

In addition to the oral care active ingredients mentioned above, other oral care active ingredients may be present in the compositions of the invention. Said oral care active ingredients may exert any beneficial effect to teeth and gums, such as reducing hypersensitivity of the teeth, reducing plaque accumulation, reducing or inhibiting demineralization and promote remineralization of the teeth, inhibiting microbial biofilm formation in the oral cavity, reducing or inhibiting gingivitis, promoting healing of sores, reducing levels of acid producing bacteria, increasing relative levels of non-cariogenic and/or non-plaque forming bacteria, reducing or inhibiting formation of dental caries, reducing, repairing or inhibiting pre-carious lesions of the enamel, treating, relieving or reducing dry mouth, cleaning the teeth and oral cavity, reducing erosion, whiten teeth, and reducing tartar build-up. Examples of said agents include alkali metal gluconate salts such as sodium gluconate, panthenol, vitamin E or a derivative thereof such as vitamin E acetate, $\alpha$-bisabolol, malic acid, calcium glycerophosphate, etc. It is to be understood that the weights of these active ingredients may vary depending on the particular active ingredient, and one of skill in the art may readily determine such amounts. Typically each oral care active ingredient is optionally present in an amount in the range from 0.001 wt% to 1 wt%%, wherein the wt% is expressed with respect to the total weight of the composition.

The oral care compositions of the present invention may comprise a pH modifying agent, which is a compound that adjust and/or maintain pH. Such agents include acidifying agents to lower pH, basifying agents to raise pH, and buffering agents to control pH within a desired range. For example, one or more compounds selected from acidifying, basifying and buffering agents can be included to provide a pH of 4.5 to 10.5, preferably 5.5 to 8.5. Any orally acceptable pH modifying agent can be used, including carboxylic, phosphoric and sulfonic acids and their salts (e.g., monopotassium phosphate, dipotassium phosphate, monosodium phosphate, trisodium phosphate, citric acid, monosodium citrate, disodium citrate, monosodium malate, etc.), alkali metal hydroxides such as sodium hydroxide, carbonates such as sodium carbonate, bicarbonates, sesquicarbonates, borates, silicates, imidazole and the like. One or more pH modifying agents are optionally present in a total amount effective to maintain the composition in an orally acceptable pH range.

The oral care compositions of the invention may optionally comprise a chelating agent, which forms complexes with metal ions, such as calcium, magnesium and iron. Examples of chelating agents are citric acid, succinic acid, gluconic acid, etidronic acid, galactaric acid, galacturonic acid, glucuronic acid, phytic acid, ethylenediaminetetraacetic acid (EDTA), methylglycine-*N*,*N*-diacetic acid (MGDA), *N*,*N*-Dicarboxymethyl glutamic acid (GLDA), and the respective salts of ammonium, sodium and potassium; poly-acrylic acids, polymers thereof and the respective salts of ammonium, sodium and potassium; and phosphoric acids and poly-phosphoric acids and the respective salts of ammonium, sodium and potassium. Preferably, the chelating agent is EDTA or an ammonium, sodium or potassium salt thereof.. One or more chelating agents are optionally present in a total amount in the range of from 0.1 to 5 wt%, wherein the wt% is expressed with respect to the total weight of the composition.

Other antimicrobial agents may also be present in the oral care compositions of the invention. Antimicrobial agents inhibit the development of microorganism in the oral care composition. Examples of suitable antimicrobial agents are chlorhexidine, triclosan, quaternary ammonium compounds (such as benzalkonium chloride) or parabens (such as

methyl or propyl paraben). One or more antimicrobial agents are optionally present in a total amount typically in the range from 0.001 wt% to 0.5 wt%, wherein the wt% is expressed with respect to the total weight of the composition.

The oral care compositions of the present invention may optionally comprise a flavoring agent that is added to modify the taste and the aroma of the oral care compositions. Flavoring agents that may be used in the compositions of the present invention include essential oils as well as various flavoring aldehydes, esters, alcohols, and similar materials. Examples of the essential oils include oils of spearmint, peppermint, aniseed, wintergreen, sassafras, clove, sage, eucalyptus, marjoram, cinnamon, lemon, lime, grapefruit, and orange. Also useful are compounds such as menthol, carvone, anethole and vainillin. Of these, the most commonly employed are the oils of peppermint and spearmint. One or more flavoring agents may be present in the oral care composition at a total concentration in the range of 0.01 wt% to 5 wt%, wherein the wt% is expressed with respect to the total weight of the composition.

The oral care composition of the invention may comprise at least one colorant, which is added to modify the colour of the compositions. Colorants herein include pigments, dyes, lakes and agents imparting a particular luster or reflectivity such as pearling agents. Any orally acceptable colorant can be used, such as titanium dioxide. One or more colorants are optionally present in a total amount in the range of from 0.0001 wt% to 5 wt%, wherein the wt% is expressed with respect to the total weight of the composition.

The oral care compositions of the invention may be in the form of mucoadhesive compositions such as a mouthwash, a toothpaste or a tooth gel.

In the context of the present invention, "mouthwash" is equivalent to "oral rinse", "mouth rinse" and "mouth bath" and designate liquid compositions which are suitable for oral hygiene, such as by reducing the microbial load in the oral cavity, in particular the bacterial plaque that causes cavities, gingivitis and bad breath. When using such compositions, they are typically maintained in the mouth passively and/or swished through the teeth by contraction of the perioral muscles and/or movement of the head, and may be gargled, and finally spit out.

In the context of the present invention, the term "toothpaste" refers to a semi solid preparation suitable for cleaning the teeth when used with a toothbrush. In the context of the present invention, the term "tooth gel" refers to a gel preparation suitable for cleaning the teeth when used with a toothbrush. Tooth gels are like toothpastes but clear or translucent in appearance. In particular, tooth gels do not comprise titanium dioxide whereas toothpastes comprise titanium dioxide.

In a particular embodiment, the oral care composition of the invention is a mouthwash and comprises water in an amount of at least 70 wt%, at least 75 wt%, or at least 80 wt%; preferably at least 75 wt% of water. The wt% is expressed with respect to the total weight of the composition.

In another particular embodiment, the oral care composition of the invention is a tooth gel or toothpaste and comprises water in an amount of at least at least 10 wt%, at least 15 wt%, at least 20 wt%, at least 25 wt%, at least 30 wt%; preferably at least 10 wt% of water. The wt% is expressed with respect to the total weight of the composition.

In another particular embodiment, the oral care composition of the invention is a tooth gel or toothpaste and comprises water in an amount of from 10 wt% to 35 wt%, from 15 wt% to 35 wt%, from 20 wt% to 35 wt%, from 25 wt% to 35 wt%, from 30 wt% to 35 wt%; preferably from 10 wt% to 35 wt% of water. The wt% is expressed with respect to the total weight of the composition.

The oral care compositions of the invention may be obtained by mixing the above mentioned ingredients.

Uses of the compositions

As explained in the background section, o-cymen-5-ol has antimicrobial properties against different bacteria and has been reported to penetrate deep-inside plaque, which helps to control the development of dental plaque, calculus formation, bleeding gums, and malodour. *o*-cymen-5-ol also reduce the breath concentrations of volatile sulphur compounds (VSC), which are associated with halitosis, as well as having direct and indirect inhibitory effects on anaerobe mediated-VSC production.

Accordingly, a second aspect of the present invention is directed to the oral care compositions of the invention for use in maintaining the health of the oral cavity, in particular for use in the treatment and/or prevention of dental plaque formation, caries formation, calculus formation, halitosis, gingivitis, periodontitis, peri-implant mucositis and/or periimplantitis.

This aspect may also be formulated as the use of an oral care composition of the invention for the manufacture of a medicament for treating and/or preventing dental plaque formation, caries formation, calculus formation, halitosis, gingivitis, periodontitis, peri-implant mucositis and/or periimplantitis.

Alternatively, this aspect may be formulated as a method for the treatment and/or prevention of dental plaque formation, caries formation, calculus formation, halitosis, gingivitis, periodontitis, peri-implant mucositis and/or periimplantitis comprising contacting a surface of the oral cavity with the oral care compositions of the invention.

Alternatively, this aspect may be formulated as the use of the oral care compositions of the invention for the treatment and/or prevention of dental plaque formation, caries formation, calculus formation, halitosis, gingivitis, periodontitis, peri-implant mucositis and/or periimplantitis.

For the above purposes, the aqueous oral composition of the invention may be contacted with a surface of the oral cavity. Said surface of the oral cavity may be selected from the group consisting of one or more teeth, surfaces of the gums, surface of the tongue and combinations thereof. For performing said contacting, when the oral care composition of the invention a mouthwash, it is typically held in the oral cavity passively or swirled around the mouth by contraction of the perioral muscles and/or movement of the head, and may be gargled, and finally spit out. In the case of mouthwashes, the amount of composition dosed in the oral cavity to perform the activity is typically from 2 to 30 ml, preferably from 5 to 20 ml and most preferable from 10 to 15 ml. The contacting may be performed for any suitable amount of time, such as for less than thirty seconds, for thirty seconds or more, for about thirty seconds, for about forty seconds, for about one minute or more. For performing the contact with a surface of the oral cavity, when the oral care composition of the invention is a tooth gel or toothpaste, it is typically applied with a toothbrush and mechanical cleaning of the teeth and/or tongue is performed, preferably for at least two minutes, more preferably for at least three minutes. In the case of tooth gels and toothpastes, the amount of composition dosed to perform the activity is typically from 0.3 to 4.0 g, preferably from 0.5 to 2.5 g. The contacting may be carried out at least once daily, preferably at least twice daily, more preferably at least three times daily, such as 1, 2, 3 or 4 times per day. The oral cavity may belong to a human or animal, preferably a human.

The term "treatment", "treating" or "treat", as used herein, refers to means reversing, alleviating, inhibiting the progress of the disease or disorder these terms refer to, or one or more symptoms of such disease or condition.

The term "prevention", "preventing" or "prevent", as used herein, refers to avoiding the appearance of a certain disease or disorder. The prevention can be complete (e.g. the total absence of a disease or disorder). The prevention can also be partial, such that for example the occurrence of a disease or disorder in a subject is less than that which would have occurred without the administration of the oral care composition of the present invention. The prevention also includes reducing the risk of suffering the disease or disorder.

The term "dental plaque" refers to a fine film comprised of remains of food, bacteria and saliva. If dental plaque is not eliminated regularly, it sticks to the surface of the enamel, attacking tooth structures and producing a series of substances that cause the gum swelling, giving rise to gingivitis (inflammation of the gum), redness and probable gingival bleeding.

The term "calculus" or "tartar" refers to a form of hardened dental plaque. It is caused by precipitation of minerals from saliva and gingival crevicular fluid in plaque on the teeth. This process of precipitation kills the bacterial cells within dental plaque, but the rough and hardened surface that is formed provides an ideal surface for further plaque formation. This leads to calculus buildup, which compromises the health of the gingiva (gums). Calculus can form both along the gumline, where it is referred to as supragingival, and within the narrow sulcus that exists between the teeth and the gingiva, where it is referred to as subgingival.

The term "gingivitis" refers to an inflammation of the gums that gives rise to an increase in sensitivity and irritation and which in many cases is accompanied by bleeding. Its most frequent trigger is dental plaque or biofilm, consisting of a fine film comprised of remains of food, bacteria and saliva. If it is not treated in time, it can evolve to periodontitis (pyorrhoea), and even enter the tooth and damage its bone structure, leading to the irreversible loss of the tooth.

The term "periodontitis" refers to an inflammatory gingival process that spreads to the periodontal ligament which holds and fixes the tooth, and even the alveolar bone, destroying both tissues. Periodontal bags appear, which are an increase in the gap or space between the surface of the tooth and the internal part of the gum. This destructive process spreads until tooth stability is compromised. Due to the loss of fixation, in the end stages the teeth become separated and loose, losing their normal location, and finally the tooth irremediably falls out. *Streptococci, spirochetes* and *bacteroides* are found to be the possible pathogens responsible for the disease [Prasanth M., Dent Res J (Isfahan), 2011, 8(2), 85-94; Menendez A. et al., Oral Microbiology Immunology, 2005, 20(1), 31-34].

The term "halitosis" or "bad breath" refers to the presence of unpleasant odours that can be detected in exhaled air. Halitosis is a condition that affects many people. There are multiple types of halitosis and they are classified depending on the source or cause of this bad breath: physiological halitosis, pathological halitosis (oral cause, 87% of all halitosis, and extraoral cause), pseudohalitosis and halitophobia. Bad breath through an oral cause is therefore the most common. This type of halitosis is caused by the production and release of volatile compounds, mainly sulphur compounds. Other molecules such as the diamines (for example, cadaverine) are also related to halitosis. The volatile sulphur compounds are caused by bacteria that grow in the mouth in conditions of oxygen absence (anaerobes). These volatile sulphur compounds come from the metabolism of food bacteria or remain trapped in the mouth.

The term "caries" or "cavities" refers to the breakdown of teeth structure due to acids produced by bacteria. *Streptococcus mutans* is considered one of the main bacteria involved in this process. In addition, other microflora like *Escherichia coli* and *Candida* are also associated with active caries lesions [Prasanth M., Dent Res J (Isfahan), 2011, 8(2), 85-94]. The cavities may be a number of different colors from yellow to black. Symptoms may include pain and difficulty with eating. Complications may include inflammation of the tissue around the tooth, tooth loss, and infection or

abscess formation. It is a disease characterized by a series of reactions that lead the teeth's hard tissues to soften, and which if it goes untreated, advances from the surface inwards, eventually leading to the irreversible destruction of the tooth. Caries normally goes unnoticed by the patient. A detailed exploration will detect stains or white chalk-like spots (areas where there is weakened enamel) or brown pigmentation on the tooth surface. This phase may be characterized by a certain sensitivity (strange sensation) to some foods, particularly sweets or hot or cold drinks, although usually there is no actual pain. When the caries advances and affects the pulp or nerve inside the tooth, pain will generally ensue, although if the affection is very slow it may destroy a large part of the nerve painlessly. When the affection is deep, abscesses may form (better known as gumboils, i.e. destroyed dental material and pus) and give rise to major inflammation and pain.

The term "peri-implant mucositis" refers to an inflammatory lesion of the soft tissues surrounding an endo-osseous implant in the absence of loss of supporting bone or continuing marginal bone loss. Peri-implant mucositis is caused by bacteria from dental biofilms, which disrupts the host-microbe homeostasis at the implant-mucosa interface, resulting in an inflammatory lesion. Biofilm removal is a prerequisite for the prevention and management of peri-implant mucositis. Peri-implant mucositis is considered a precursor for peri-implantitis.

The term "periimplantitis" refers to an inflammatory lesion of the mucosa that affects the surrounding tissue of the implant, predominately the supporting bone with loss of osseointegration. As peri-implant mucositis, it is also caused by the adhesion of pathogenic bacterial biofilms on the implant surface and peri-implant tissues.

In the context of the invention, the term "about" when characterizing a value refers to $\pm$10% of said value, preferably $\pm$5% of said value.

Preferably, the expression "one or more" refers to 1, 2, 3, 4, 5, 6, 7, 8, 9 or 10, more preferably to 1, 2, 3, 4 or 5, even more preferably to 1, 2 or 3.

The following examples are provided to illustrate the invention but should not be considered as limiting its scope.

## Examples

Compositions

The mouthwash compositions of comparative examples 1 to 3 and of example 4 are prepared using the procedure detailed below:
In a reactor water is added followed by colorants, xylitol, sucralose and vainillin, the mixture was stirred for 15 minutes.

In a separate first mixing vessel the cellulosic polymer (such as hydroxypropyl methyl cellulose or hydroxyethyl cellulose) is dispersed in glycerine. This dispersion is added to the reactor and stirred.

In a second mixing vessel a mixture of propylenglycol, PEG-40 hydrogenated castor oil, menthol, neohesperidin DC, aroma and o-cymen-5ol is prepared under stirring until complete dissolution. The mixture of the second mixing vessel is then added to the content of the reactor mixing vessel and is stirred for 30 minutes.

A 20% aqueous solution of chlorhexidine digluconate is added to the reactor and is stirred for 30 minutes at high speed. The pH is then adjusted to 5.4-6.0 using sodium citrated and citric acid as needed and stirring for 20 minutes. The resulting mixture is filtered through a 5$\mu$m filter.

The mouth wash compositions of comparative examples 1, 2 3 and 5 and examples 4 and 6 shown below do all comprise the following ingredients in common accounting for 18.3884 wt.% of the total compositions:

Xylitol 1 wt.%
Sucralose 0.13 wt.%
Vainillin 0.003 wt.%
Glycerin 8 wt.%
Neohesperidin DC 0.003 wt.%
Sodium citrate 0.05 wt.%
Citric acid monohydrate 0.0112 wt.%
Propylenglycol 8 wt.%
PEG-40 hydrogenated castor oil 1 wt.%
Flavour (1/074569) 0.11 wt.%
Menthol 0.08 wt.%
Colorant (80% E-124) 0.0012 wt.%

In addition, the compositions of comparative examples 1, 2, 3 and 5 and examples 4 and 6 contain the ingredients specified in table 1 below. The ensemble of the ingredients mentioned in the previous paragraph are is indicated in the table as "base composition".

Table 1

| *Ingredient* | *Comp. Ex. 1* | *Comp. Ex. 2* | *Comp. Ex. 3* | *Ex. 4* | *Comp. Ex. 5* | *Ex. 6* |
|---|---|---|---|---|---|---|
| Base composition | 18.3884 | 18.3884 | 18.3884 | 18.3884 | 18.3884 | 18.3884 |
| Chlorhexidine digluconate (20%) | 0 | 0 | 0.636 | 0.636 | 0.636 | 0.636 |
| *o*-Cymen-5-ol | 0 | 0.1 | 0 | 0.1 | 0 | 0.1 |
| Hydroxypropyl methyl cellulose (BENECEL KM4 Pharma CR) | 0.10 | 0.10 | 0.10 | 0.10 | - | - |
| Hydroxy ethyl cellulose (NATROSOL M PHARMA) | - | - | - | - | 0.10 | 0.10 |
| Water | *q.s.* 100 | *q.s.* 100 | *q.s.* 100 | *q.s.* 100 | *q.s.* 100 | *q.s.* 100 |

The gel compositions of comparative examples 7 to 9 and of example 10 are prepared using the procedure detailed below:
Sorbitol and glycerine are added to a reactor. Then hydroxypropyl methyl cellulose is added gradually through the reactor mouth. The mixture is stirred with the paddles and the vacuum connected at a speed of 50 rpm for 10 minutes. After the 10 minutes of homogenization, the total of purified water, sucralose and vanillin are added. The paddle stirrer is the switched on at a speed of 50 rpm and the vacuum and turrax are switched on at 8000 rpm for 30 minutes. After this time, chlorhexidine digluconate and a premix prepared with propylene glycol, flavour, PEG 40 hydrogenated castor oil, menthol and o-cymen-5-ol are added. The mixture is the homogenised with paddle stirring at a speed of 50 rpm and the vacuum and turrax are switched on at a speed of 8000 rpm for 20 minutes.

The gel compositions of comparative examples 7 to 9 and example 10 shown below do all comprise the following ingredients in common accounting for 24.593 wt.% of the total compositions:

Propylenglycol 10 wt. %
Glycerin 7 wt. %
Sorbitol 7 wt.
PEG 40 hydrogenated castor oil 0.3 wt.%
Vainillin 0.003 wt. %
Menthol 0.1 wt. %
Flavour (1/074569) 0.12 wt. %
Sucralose 0.07 wt. %

In addition, the compositions of comparative examples 7 to 9 and example 10 contain the ingredients specified in table 2 below. The ensemble of the ingredients mentioned in the previous paragraph are is indicated in the table as "base composition".

Table 2

| *Ingredient* | *Comparative example 7* | *Comparative example 8* | *Comparative example 9* | *Example 10* |
|---|---|---|---|---|
| Base composition | 24.593 | 24.593 | 24.593 | 24.593 |
| Chlorhexidine digluconate (20%) | 0 | 1.060 | 0 | 1.060 |
| *o*-Cymen-5-ol | 0 | 0 | 0.1 | 0.1 |
| Hydroxypropyl methyl cellulose (BENECEL KM4 Pharma CR) | 3.2 | 3.2 | 3.2 | 3.2 |
| Water | *q.s.* 100 | *q.s.* 100 | *q.s.* 100 | *q.s.* 100 |

Determination of the mucoadhesion capacity of formulations to the oral mucosa

The method described is based on the mucin adhesion model which is described, for example, in Madsen, F. et al. Journal of Controlled Release 50 (1998) 167-178 to assess the interaction between a given composition and mucin, which is a major component of the mucus of the oral mucose. This interaction is quantified by a relative viscosity measurement ($\eta_{REL}$).

**Materials**

1. Preparation of a 10% mucin dispersion

5 grams of mucin type III are weighed in a beaker. Water is then added to a final weight of 50 grams. The beaker is stirred to complete dispersion until no lumps remain. The pH is adjusted with 0,5N NaOH or 1N HCl depending on whether pH is to be raised or lowered, to a final pH of 6.2 and 7.0.

2. Preparation of a 6% mucin dispersion

18 grams of the above mentioned 10% mucin dispersion are weighed in a beaker. Water is then added to a final weight of 30 grams. The beaker is stirred to complete dispersion until no lumps remain.

3. Preparation of a 3% mucin dispersion (M)

Note: prepare the sample in duplicate
3 grams of the above mentioned 6 % mucin dispersion into a beaker. Water is then added to a final weight of 6 grams. The beaker is stirred for at least 30 minutes to complete homogenization.

4. Preparation of a dispersion of the composition to be tested (P)

Note: prepare the sample in duplicate
3 grams of the composition to be tested are weighed in a beaker. Water is then added to a final weight of 6 grams. The beaker is stirred for at least 30 minutes to complete homogenization.

5. Preparation of mixture of mucin and the composition to be tested (P+M)

Note: prepare the sample in duplicate
3 grams of the 3% mucin dispersion are weighed in a beaker. 3 grams of the composition to be tested are added. The beaker is stirred for at least 30 minutes to complete homogenization.

**Testing process**

Using a Haake Mars viscosimeter viscosities at a shear rate of 0.2 $s^{-1}$ (for highly viscous products) or at a shear rate of 100 $s^{-1}$ (for non-highly viscous products) are calculated by building a line of viscosity vs. shear rate and interpolating the viscosity values ($\eta_{P+M}$, $\eta_P$ and $\eta_M$) at a shear rate of 0.2 $s^{-1}$ (for highly viscous products) or 100 $s^{-1}$ (for non-highly viscous products) .

The line of viscosity vs. shear rate is made by measuring viscosity (two duplicates for each duplicate sample) employing the following conditions:

| | |
|---|---|
| Stabilisation 1: | 30 s at 0 $s^{-1}$ |
| Stabilisation 2: | 30 s at 0.1 $s^{-1}$ |
| Temperature: | 37°C |

Measurements of viscosity are made at the following shear rates which maintained during 30 s before taking the viscosity measure.

Shear rate 0.1 $s^{-1}$
Shear rate 1.0 $s^{-1}$
Shear rate 10.0 $s^{-1}$

Shear rate 100.0 $s^{-1}$
Shear rate 1000.0 $s^{-1}$

Note:

To discriminate between highly viscous products and non-highly viscous products a first viscosity measurement at a shear rate of 100 $s^{-1}$ is carried out using a plate 35 sensor and a 0.3 mm groove.

When the viscosity of the formula to be tested measured at the above-mentioned conditions is higher than 150 mPa·s the product is considered a highly viscous product and the following conditions are used to build the viscosity/shear rate line and to interpolate the viscosity values at a shear rate of 0.2 $s^{-1}$:

Sensor: Plate 35 or equivalent
Groove: 0.3 mm

When the viscosity of the formula to be tested measured at the above-mentioned conditions is lower 150 mPa's the product is considered a non-highly viscous product and following conditions are used to build the viscosity/shear rate line and to interpolate the value at a shear rate of 100 $s^{-1}$::

Sensor: 35/2° cone or equivalent

**Calculations**

From the viscosity readings ($\eta_{P+M}$, $\eta_P$ and $\eta_M$) obtained from each sample (4 readings for each sample) of the mucin dispersion P, the compositions to be tested M and the mixture of mucin dispersion and composition to be tested (P+M) an average is taken and the following calculations are made to obtain the value $\eta$-relative and to be able to define the degree of mucoadhesivity.

$$\eta_{REL} = \eta_{P+M}/(\eta_P + \eta_M)$$

wherein $\eta_{P+M}$ is the viscosity of the mixture of the product to be tested with the 3% mucin dispersion, $\eta_P$ is the viscosity of the product to be tested and $\eta_M$ is the viscosity of the 3% mucin dispersion.

The higher the value of $\eta_{REL} \geq$ the stronger is the interaction between mucin and the product under study. The expected in-vivo mucoadhesivity of the composition will be higher the higher the $\eta_{REL}$ value.

**Results**

The mucoadhesive behavior of the compositions of comparatives examples 1-3, 5 and 7-9 and that of the compositions of examples 4, 6 and 10 determined using the above-describes methodology is reported in tables 3 and 4 below.

Table 3

| *Ingredient* | *Comp. Ex. 1* | *Comp. Ex. 2* | *Comp. Ex. 3* | *Ex. 4* | *Comp. Ex. 5* | *Ex. 6* |
|---|---|---|---|---|---|---|
| $\eta$-relative | 1.15 | 1.05 | 0.96 | 1.19 | 1.10 | 1.12 |

Table 4

| *Ingredient* | *Comp. Ex. 7* | *Comp. Ex. 8* | *Comp. Ex. 9* | *Ex. 10* |
|---|---|---|---|---|
| $\eta$-relative | 1.15 | 1.05 | 0.96 | 1.19 |

The results summarized in the tables above show an unexpectedly good mucoadhesive behavior of the compositions of the invention (examples 4, 6 and 10) when compared with similar compositions lacking at least one of the following ingredients: a) cellulosic polymer, b) *o*-cymen-5-ol, and (c) chlorhexidine or salts thereof (comparative examples 1-3, 5 and 7-9).

**Claims**

1. Oral care composition comprising:

    - a cellulosic polymer,
    - *o*-cymen-5-ol, and
    - chlorhexidine or salts thereof.

2. Oral care composition according to claim 2, wherein the cellulosic polymer is selected from the group consisting of hydroxypropyl methylcellulose and hydroxyethyl cellulose.

3. Oral care composition according to any one of the preceding claims wherein the cellulosic polymer is present at a concentration of from 0.05 to 4 wt% with respect to the total weight of the composition.

4. Oral care composition according to any one of the preceding claims wherein o-cymen-5-ol is present at a concentration of from 0.005 to 1 wt%, preferably from 0.01 to 0.5 wt%, more preferably from 0.02 to 0.2 wt%, most preferably from 0.05 to 0.1 wt% with respect to the total weight of the composition.

5. Oral care composition according to any one of the preceding claims wherein chlorhexidine or the salts thereof are present at a concentration of from 0.05 to 0.5 wt% (expressed as chlorhexidine free base) with respect to the total weight of the composition.

6. Oral care composition according to any one of the preceding claims not including any zinc salt having a solubility in water higher than $3.0 \cdot 10^4$ mg/L at 25 °C.

7. Oral care composition according to any one of the preceding claims not including any glycyrrhizin derivative.

8. Oral care composition according to any one of the preceding claims not including hydroxyacetophenone.

9. Oral care composition according to any one of the preceding claims comprising water.

10. Oral care composition according to claim 9, wherein water is present in an amount of at least 10 wt%, at least 15 wt%, at least 20 wt%, at least 25 wt%, at least 30 wt%, at least 40 wt%, at least 50 wt%, at least 60 wt%, at least 70 wt%, at least 75 wt%, or at least 80 wt%, wherein the wt% is expressed with respect to the total weight of the composition.

11. Oral care composition according to any of the preceding claims further comprising one or more ingredients selected from the group consisting of fluoride ion sources, surfactants, co-solvents, humectants, sweeteners, binders, mouthfeel agents, abrasives, desensitizing agents, other oral care active ingredients, pH modifying agents, antimicrobial agents other than chlorhexidine or salts thereof, chelating agents, flavoring agents, and colorants.

12. Oral care composition according to any of the preceding claims in the form of a mouthwash, a tooth gel or a toothpaste.

13. Oral care composition according to any of the preceding claims in the form of a mouthwash which comprises:

    - 0.005 to 1 wt% of o-cymen-5-ol,
    - 0.05 to 4 wt% of a cellulosic polymer selected from the group consisting of hydroxypropyl methyl cellulose and hydroxyethyl cellulose,
    - 0.05 to 4 wt% of chlorhexidine or a salt thereof (expressed as chlorhexidine free base)
    - at least 70 wt% of water,
    - optionally 5 to 10 wt% of propylene glycol,
    - optionally 1 to 10 wt% of glycerin, and
    - optionally 1 to 5 wt% PEG40 hydrogenated castor oil,

    wherein the wt% is expressed with respect to the total weight of the composition.

14. Oral care composition according to any of claims 1 to 12 in the form of a tooth gel or toothpaste which comprises:

- - 0.005 to 1 wt% of o-cymen-5-ol,

  - 0.05 to 4 wt% of a cellulosic polymer selected from the group consisting of hydroxypropyl methyl cellulose and hydroxyethyl cellulose,
  - 0.05 to 4 wt% of chlorhexidine or a salt thereof (expressed as chlorhexidine free base)

- - 10 to 75 wt% of water,
- - optionally 5 to 55 wt% of sorbitol,
- - optionally 0.1 to 1.5 wt% xanthan gum,
- - optionally 0.5 to 10 wt% of propylene glycol, and
- - optionally 1 to 35 wt% of glycerin,
- wherein the wt% is expressed with respect to the total weight of the composition.

**15.** Oral care composition according to any of the preceding claims for use in the treatment and/or prevention of dental plaque formation, caries formation, calculus formation, halitosis, gingivitis, periodontitis, peri-implant mucositis and/or periimplantitis.

Europäisches Patentamt
European Patent Office
Office européen des brevets

# EUROPEAN SEARCH REPORT

Application Number
EP 22 38 3254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2000 319153 A (LION CORP) 21 November 2000 (2000-11-21) * paragraphs [0001], [0002], [0032]; example 2 * * paragraphs [0013], [0017] * | 1-15 | INV. A61Q11/00 A61K9/00 A61K8/73 A61K8/58 A61K8/34 A61K8/60 A61K8/365 A61K8/92 A61K8/02 A61K8/04 A61K47/10 A61K47/12 A61K47/24 A61K47/26 A61K47/38 A61K47/44 A61P1/02 |
| X | JP H07 72125 B2 (LION CORP) 2 August 1995 (1995-08-02) * paragraphs [0001], [0002]; claim 2; example 6 * * example 11 * | 1-15 | |
| X | JP S59 101417 A (LION CORP) 12 June 1984 (1984-06-12) * paragraphs [0001], [0002]; example 6 * | 1-15 | |
| X | JP H07 48237 A (KAO CORP) 21 February 1995 (1995-02-21) * paragraph [0001]; example 4 * * examples 5,6 * | 1-15 | TECHNICAL FIELDS SEARCHED (IPC) A61K A61Q A61P |

The present search report has been drawn up for all claims

| Place of search | Date of completion of the search | Examiner |
|---|---|---|
| Munich | 9 May 2023 | Cetinkaya, Murat |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 22 38 3254

This annex lists the patent family members relating to the patent documents cited in the above-mentioned European search report. The members are as contained in the European Patent Office EDP file on
The European Patent Office is in no way liable for these particulars which are merely given for the purpose of information.

09-05-2023

| Patent document cited in search report | Publication date | Patent family member(s) | Publication date |
|---|---|---|---|
| JP 2000319153 A | 21-11-2000 | NONE | |
| JP H0772125 B2 | 02-08-1995 | JP H0772125 B2 | 02-08-1995 |
| | | JP S6360921 A | 17-03-1988 |
| JP S59101417 A | 12-06-1984 | JP H0347245 B2 | 18-07-1991 |
| | | JP S59101417 A | 12-06-1984 |
| JP H0748237 A | 21-02-1995 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005014047 A1

### Non-patent literature cited in the description


- **MAŠKOVÁ E. et al.** *Journal of Controlled Release,* 10 August 2020, vol. 324, 695-727
- **PARK, Y.-D. et al.** *Int. J. Clin. Prev. Dent.,* 2010, vol. 6 (2), 55-61
- **PIZZEY, R. L.** *Int. Dent. J.,* 2011, vol. 61 (3), 33-40
- **NAJAFI MH et al.** *Dent Res J,* 2012, vol. 9, 305-8
- **PRASANTH M.** *Dent Res J (Isfahan),* 2011, vol. 8 (2), 85-94
- **MENENDEZ A. et al.** *Oral Microbiology Immunology,* 2005, vol. 20 (1), 31-34
- **MADSEN, F. et al.** *Journal of Controlled Release,* 1998, vol. 50, 167-178